# EUROPEAN PATENT APPLICATION

(11) **EP 4 461 247 A1**
(43) Date of publication of application: **13.11.2024**
(21) Application number: 24174515.7
(22) Date of filing: 07.05.2024
(51) Int. Cl.: A61B 18/14, A61B 18/00, A61B 18/12

(54) **ELECTRICAL CONNECTOR FOR A CATHETER DEVICE**

(30) Priority: 08.05.2023 US 202318313708
(71) Applicant: Biosense Webster (Israel) Ltd., 2066717 Yokneam (IL)
(72) Inventor: RODRIGUEZ SOTO, Juan, Irvine, 92618 (US); EBRAHIMI, Babak, Irvine, 92618 (US); ABBAS, Mohammad, Irvine, 92618 (US); VAN NIEKERK, Pieter Emmelius, Irvine, 92618 (US); BASU, Shubhayu, Irvine, 92618 (US)
(74) Representative: Carpmaels & Ransford LLP

(57) **Abstract**

The disclosed technology includes an electrical connector for a catheter device comprising a body extending along a longitudinal axis from a proximal portion to a distal portion, the proximal portion configured to be attached to a tubular shaft and the distal portion configured to be attached to an end effector. The electrical connector can comprise a plurality of proximal electrical pads disposed on the body at the proximal portion, the proximal electrical pads configured to be in electrical communication with a plurality of tubular shaft electrical pads. The electrical connector can comprise a plurality of distal electrical pads disposed on the body at the distal portion, the distal electrical pads configured to be in electrical communication with a plurality of end effector electrical pads. The electrical connector can comprise a plurality of electrical traces. Each electrical trace can connect a respective proximal electrical pad to a respective distal electrical pad.

## Description

### Field

The present invention relates generally to medical devices comprising an end effector with electrodes, and further relates to, but not exclusively, to electrical connectors for such medical devices.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Regions of cardiac tissue can be mapped by a medical probe to identify the abnormal electrical signals. The same or different medical probe can be used to perform ablation. Some example probes include a number of spines with electrodes positioned thereon. The electrodes are generally attached to the spines and the spines are configured to extend outwardly when deployed from a sheath. The electrodes are then brought into contact with tissue for mapping or ablation of the tissue.

Various different catheter designs exist to achieve mapping or ablation of cardiac tissues in many different places in the heart. For example, some catheters include end effectors having spines arranged in a planar array while other end effectors are designed with spines configured to bow radially outward when deployed from the sheath. Further, each different end effector design can have a different number of electrodes with the number of electrodes ranging from one electrode to hundreds of electrodes. As will be appreciated, as the number of electrodes increases for a given end effector, so does the number of electrical wires or traces that must be connected to each end effector. Traditionally, catheters are made with individual wires running to each end effector with those individual wires extending through the catheter shaft all the way to the handle, making manufacturing of the catheters difficult and time consuming. What is needed, therefore, is a device and method for reducing the complexity, time, and associate cost of manufacturing a medical probes having different numbers of electrodes disposed on the end effector. These and other problems can be addressed by the technology disclosed herein.

### SUMMARY

There is provided, in accordance with the disclosed technology, an electrical connector for a catheter device. The electrical connector can comprise a body extending along a longitudinal axis from a proximal portion to a distal portion. The proximal portion can be configured to be attached to a tubular shaft and the distal portion can be configured to be attached to an end effector. The electrical connector can comprise a plurality of proximal electrical pads disposed on the body at the proximal portion. The proximal electrical pads can be configured to be in electrical communication with a plurality of tubular shaft electrical pads. The electrical connector can comprise a plurality of distal electrical pads disposed on the body at the distal portion. The distal electrical pads can be configured to be in electrical communication with a plurality of end effector electrical pads. The electrical connector can comprise a plurality of electrical traces. Each electrical trace can connect a respective proximal electrical pad to a respective distal electrical pad.

The disclosed technology can include a medical probe comprising a tubular shaft extending along a longitudinal axis of the medical probe, an end effector comprising a plurality of electrodes, and an electrical connector disposed between the tubular shaft and the end effector.

The electrical connector can comprise a body extending along the longitudinal axis from a proximal portion to a distal portion, a plurality of proximal electrical pads disposed on the body at the proximal portion, a plurality of distal electrical pads disposed on the body at the distal portion, and a plurality of electrical traces. The proximal electrical pads can be configured to align with, and electrically connect to, a plurality of tubular shaft electrical pads disposed on the tubular shaft. The distal electrical pads can be configured to align with, and electrically connect to, a plurality of end effector electrical pads disposed on the end effector. Each electrical trace can connect a respective proximal electrical pad to a respective distal electrical pad.

### BRIEF DESCRIPTION OF THE DRAWINGS

The present disclosure will be more fully understood from the following detailed description of embodiments thereof, taken together with the drawings, in which:
FIG. 1 is a schematic pictorial illustration of a medical system including a medical probe with a distal end comprising electrodes, in accordance with the disclosed technology;
FIG. 2 is a schematic pictorial illustration of an end effector, electrical connector assembly, and handle, in accordance with the disclosed technology;
FIG. 3 is an exploded view of the tubular shaft, electrical connector, and the end effector, in accordance with the disclosed technology;
FIG. 4A is an exploded section view of the tubular shaft, electrical connector, and the end effector, in accordance with the disclosed technology;
FIG. 4B is a section view of a partially assembled view of the tubular shaft, electrical connector, and the end effector, in accordance with the disclosed technology;
FIG. 5A is a perspective view of a proximal portion of an end effector, in accordance with the disclosed technology;
FIG. 5B is a top view of a proximal portion of an end effector, in accordance with the disclosed technology;
FIG. 6A is a perspective view of an electrical connector, in accordance with the disclosed technology;
FIG. 6B is a first side view of the electrical connector, in accordance with the disclosed technology;
FIG. 6C is a second side view of the electrical connector, in accordance with the disclosed technology;
FIG. 7A is a perspective view of a distal end of a catheter shaft, in accordance with the disclosed technology;
FIG. 7B is a top view of the distal end of the catheter shaft, in accordance with the disclosed technology;
FIG. 8 is a perspective exploded view of another example tubular shaft, electrical connector, and the end effector, in accordance with the disclosed technology;
FIG. 9A is a perspective view of an electrical wire and connection pad, in accordance with the disclosed technology;
FIG. 9B is a perspective view of the electrical wire connected to the electrical connector, in accordance with the disclosed technology;
FIG. 10A is an example planar end effector, in accordance with the disclosed technology;
FIG. 10B is an example of a circular end effector, in accordance with the disclosed technology;
FIG. 10C is an example basket catheter end effector, in accordance with the disclosed technology;
FIG. 10D is an example end effector having a plurality of arms, in accordance with the disclosed technology; and
FIG. 11 is an exploded view of another example of the electrical connector assembly, in accordance with the disclosed technology.

### DETAILED DESCRIPTION

The disclosed technology relates to an electrical connector that can be used with multiple different end effectors and catheter shafts. The electrical connector includes electrical connection points on the proximal side and the distal side that can be configured to align with corresponding electrical connection points on the catheter shaft and the end effector. In this way, the electrical connector can make it possible for multiple different end effectors to be used with the same catheter shaft. For example, a given catheter shaft can be configured to be used with a plurality of end effectors, with each end effector having a different number of electrodes, different configuration of spines, and or a different end use (e.g., mapping or ablation). The electrical connector can be positioned between a chosen end effector and the catheter shaft and be configured to align and electrically connect the end effector with the catheter shaft. In this way, the electrical connector can greatly simplify the manufacturing process by eliminating the requirement that each catheter shaft be made specifically for each end effector.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to an ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, method or uses and devices that can be used for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue to generate an ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

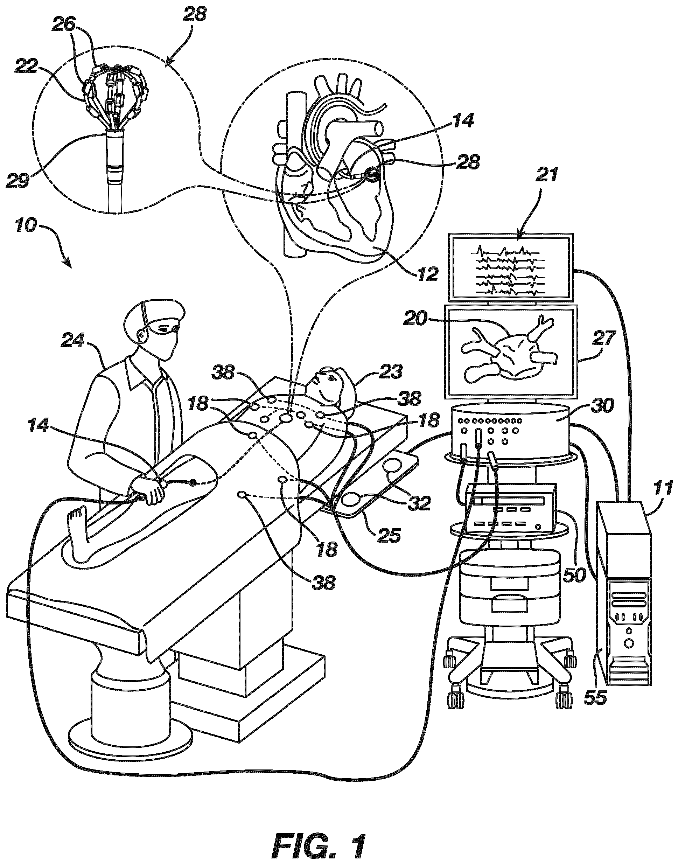

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing and ablating is illustrated herein. Physician 24 brings a distal tip 28 (sometimes referred to herein as end effector 28) of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at distal tip 28 and configured to sense the IEGM signals and/or to ablate tissue. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, the entireties of each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, the entireties of each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

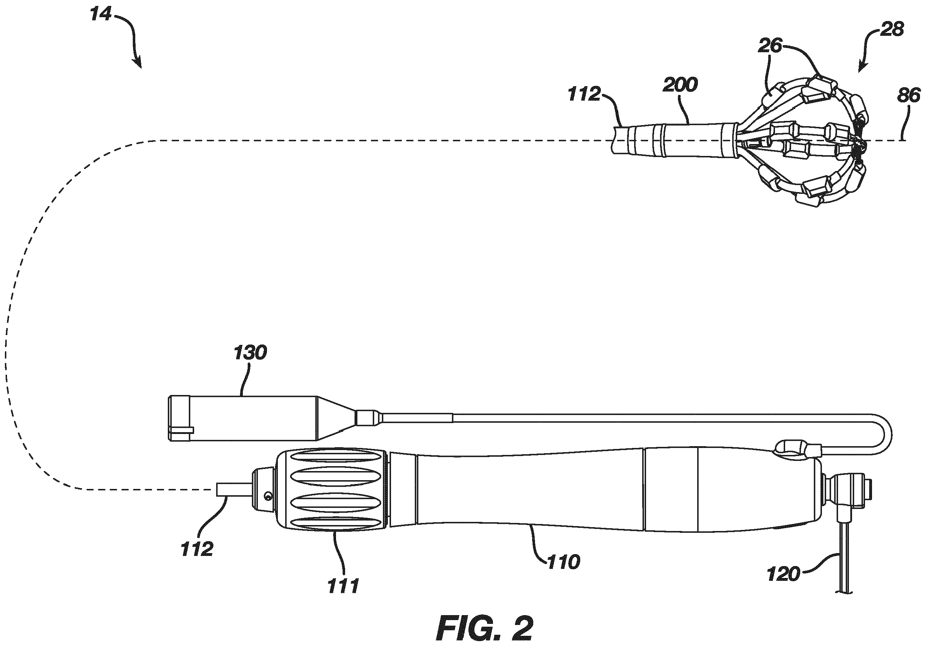

FIG. 2 is a schematic pictorial illustration of an end effector 28, electrical connector assembly 200, and handle 110, in accordance with the disclosed technology. The components shown in FIG. 2 are not drawn to scale, with emphasis instead being on the various components shown. As shown in FIG. 2, the catheter 14 can include a handle 110, a tubular shaft 112, an electrical connector 200, and an end effector 28. As a non-limiting example, the end effector 28 can be inserted into a heart 12 of a patient by being inserted through a delivery sheath. The catheter 14 can be configured such that the end effector 28 can be steered by manipulation of the handle 110 and/or by an actuator 111 disposed on the handle 110. The handle 110 can further include an irrigation supply line 120 and an electrical connection point 130. The irrigation supply line 120 can be connected to an irrigation supply and be configured to deliver irrigation fluid to the end effector 28. The electrical connection point 130 can be or include an electrical plug or other components that allow the catheter 14 to be connected to the PIU 30 to receive and/or to transmit electrical signals to the electrodes 26 on the end effector 28.

The end effector 28 shown in FIG. 2 illustrates a basket catheter having a plurality of spines 22 bowing radially outward from a longitudinal axis 86. A plurality of electrodes 26 can be attached to the spines 22 with the electrodes 26 being configured for detecting electrophysiological signals and/or for delivering ablative energy to tissues. The end effector 28 shown in FIG. 2, however, is offered for illustrative purposes and, as will become apparent throughout this disclosure, various other types of end effectors 28 can be used in place of the basket catheter shown in FIG. 2.

An electrical connector 200 can be disposed between the tubular shaft 112 and the end effector 28. The electrical connector 200 can be configured to electrically connect the electrodes 26 to the tubular shaft 112 and the electrical connection point 130 and, ultimately, with the PIU 30 to receive and/or transmit electrical signals to or from the electrodes 26. As will be described in greater detail herein, the electrical connector 200 can be configured to be used with multiple different types of end effectors 28 and tubular shafts 112. In some examples, the tubular shaft 112 can be a universal tubular shaft 112 having multiple wires or electrical traces extending therethrough and having electrical pads disposed at the distal end for connecting to the electrical connector 200. The tubular shaft 112 can be configured such that it can be used with multiple different types of end effectors 28 and the electrical connector 200 can act as an adapter between the tubular shaft 112 and the multiple different types of end effector 28.

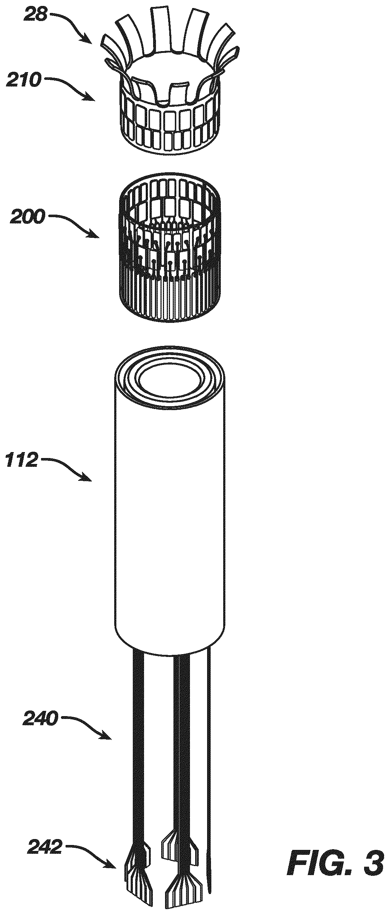

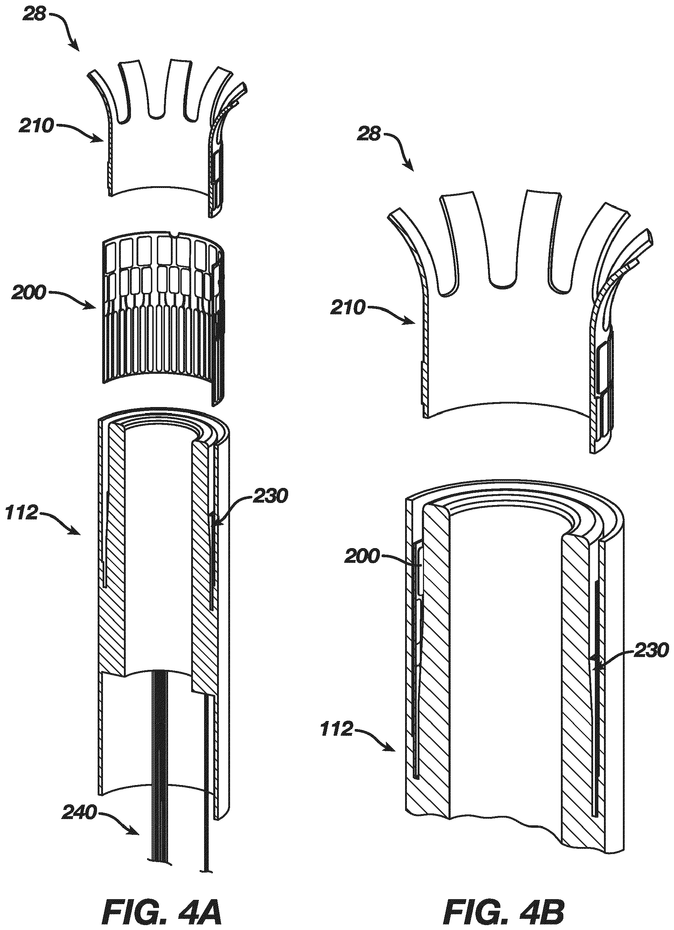

FIG. 3 is an exploded view showing the electrical connector 200 with a distal end of the tubular shaft 112 and a proximal end of the end effector 28, in accordance with the disclosed technology. For the sake of simplicity, only a portion of the tubular shaft 112 and the end effector 28 are shown. As shown, the electrical connector 200 can be disposed between the distal end of the tubular shaft 112 and the proximal end of the end effector 28. The end effector 28 can have an end effector electrical connection assembly 210 configured to physically and electrically connect to the electrical connector 200. Similarly, as shown in FIG. 4A and as will be described in greater detail herein, the distal end of the tubular shaft 112 can have a tubular shaft connection assembly 230 configured to physically and electrically connect to the electrical connector 200. FIG. 3 further illustrates electrical traces 240 that extend through the tubular shaft 112 and can include termination points 242 that can be configured to electrically connect with the handle 110.

Although the electrical connector 200 is shown as being generally cylindrical throughout the figures, it will be appreciated that the electrical connector 200 can comprises various other shapes other than that shown. For example, the electrical connector 200 can be a rectangular prism, a pentagonal prism, a hexagonal prism, comprise a star cross-sectional shape, etc. Furthermore, the electrical connector 200 can be configured to taper from one end to the other end (e.g., taper from the proximal end to the distal end, and vice-versa), taper at both ends, etc. Accordingly, the disclosed technology should not be construed as being limited to the cylinder shape as shown in the figures.

FIG. 4A is an exploded section view of the tubular shaft 112, electrical connector 200, and the end effector 28 while FIG. 4B is a partially-assembled view of the same components, in accordance with the disclosed technology. As shown, the electrical connector 200 can be configured to fit in the proximal end of the tubular shaft 112 and be in electrical communication with the tubular shaft connection assembly 230. Similarly, although not shown, the end effector electrical connection assembly 210 can be configured to fit in the proximal end of the tubular shaft 112 and be in electrical communication with the electrical connector 200. When fully assembled, the electrical connector 200 can facilitate electrical connection between the tubular shaft connection assembly 230 and the end effector electrical connection assembly 210.

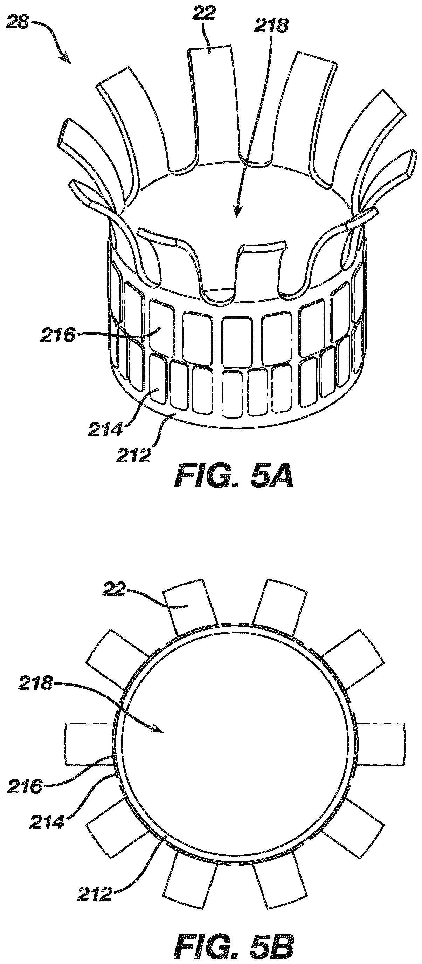

FIG. 5A is a perspective view of a proximal portion of an end effector 28, in accordance with the disclosed technology. For the sake of simplicity, only a portion of the end effector 28 is shown. As will be appreciated by one of skill in the art, the end effector 28 shown in FIG. 5A can represent a proximal portion of a basket assembly with a plurality of spines 22 and a plurality of electrodes. As shown in FIG. 5A, the end effector 28 can include proximal hub 212 having a plurality of first end effector electrical pads 214 and a plurality of second end effector electrical pads 216. The first end effector electrical pads 214 and the second end effector electrical pads 216 can each be electrically connected to respective electrodes 26 disposed on the spines 22. Alternatively, or in addition, at least some of the first end effector electrical pads 214 or the second end effector electrical pads 216 can be in electrical communication with other sensors or devices disposed on the end effector 28 such as position sensors, temperature sensors, etc. In some examples, the first end effector electrical pads 214 can be electrically connected to a plurality of mapping electrodes while the second end effector electrical pads 216 can be electrically connected with a plurality of ablation electrodes, or vice-versa. As will be appreciated, the first end effector electrical pads 214 and the second end effector electrical pads 216 can be arranged and configured according to the particular application of the end effector 28. Thus, although the end effector 28 is shown as being a basket catheter, it will be appreciated that other types of end effectors can take the place of the basket catheter shown.

FIG. 5B is a top view of a proximal portion of the end effector 28 of FIG. 5A, in accordance with the disclosed technology. As shown, the proximal hub 212 can have a lumen 218 extending therethrough. In some examples, the end effector 28 can have an irrigation hub, push rod, or various other components of the end effector 28 extending through the lumen 218.

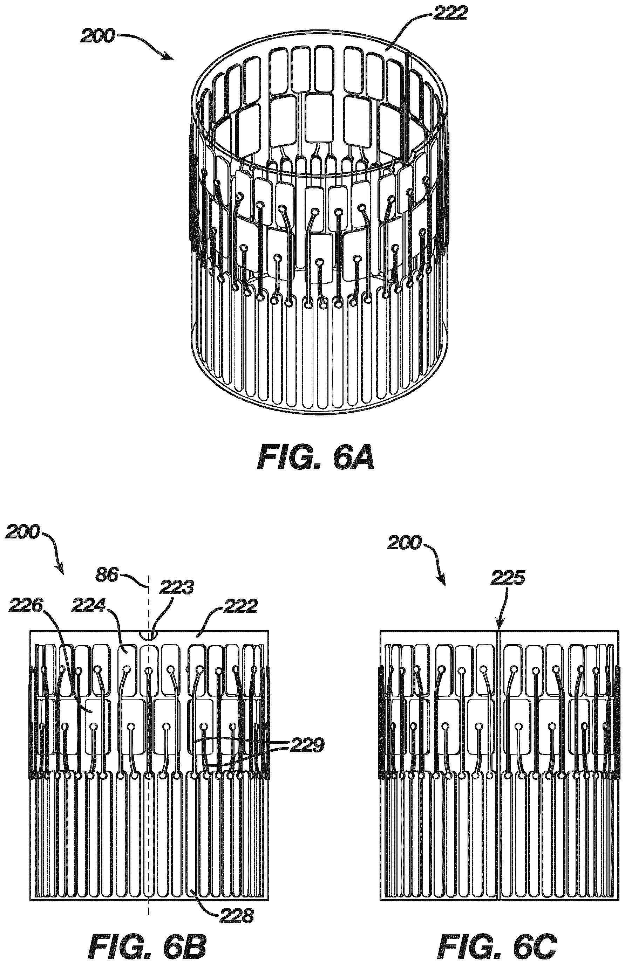

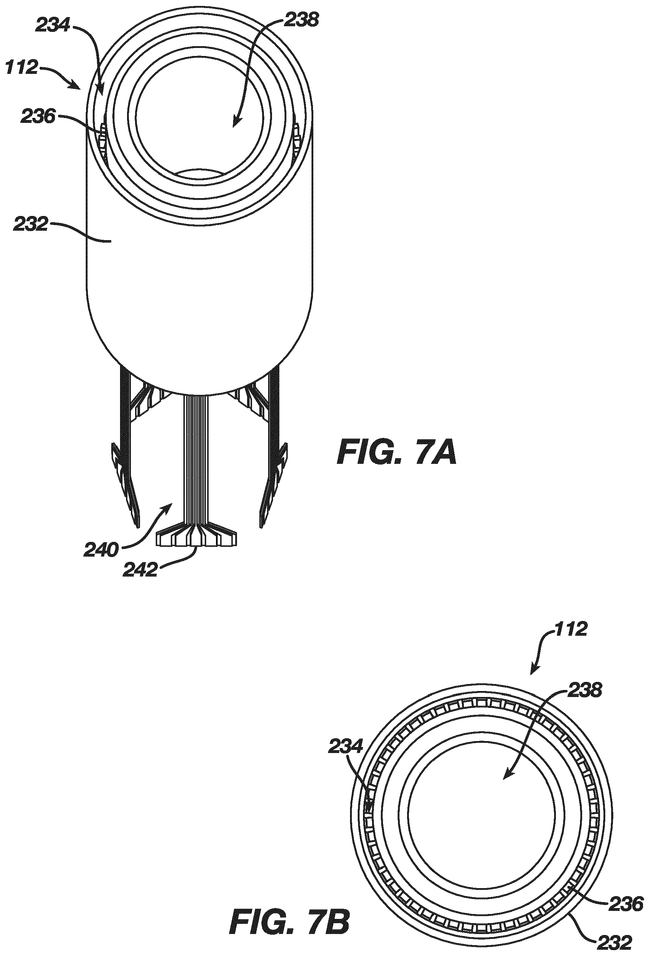

FIG. 6A is a perspective view of an electrical connector 200, in accordance with the disclosed technology. The electrical connector 200 can include a body 222 having a plurality of proximal electrical pads 228 disposed at a proximal end of the body 222 and configured to contact, and be in electrical communication with, the tubular shaft electrical pads 236 (as shown in FIGs. 7A and 7B) when assembled. The electrical connector 200 can further include a plurality of first distal electrical pads 224 and a plurality of second distal electrical pads 226 disposed at a distal end of the body 222 and configured to contact and be in electrical communication with the first and second end effector electrical pads 214, 216. The proximal electrical pads 228 can each be electrically connected to the first distal electrical pads 224 or the second distal electrical pads 226 via a plurality of electrical traces 229 (or wires). The plurality of first distal electrical pads 224, the plurality of second distal electrical pads 226, and the plurality of proximal electrical pads 228 can be disposed radially as well as longitudinally around the electrical connector 200.

The proximal electrical pads 228, the first distal electrical pads 224, and the second distal electrical pads 226 can each be disposed on an inwardly-facing surface or an outwardly-facing surface of the body 222. Alternatively, only some of the proximal electrical pads 228, the first distal electrical pads 224, or the second distal electrical pads 226 can be disposed on the inwardly-facing surface of the body 222 while the remainder can be disposed on the outwardly-facing surface of the body 222. It will be appreciated by one of skill in the art that the proximal electrical pads 228, the first distal electrical pads 224, and the second distal electrical pads 226 can be positioned on either surface to correspond with the arrangement of the configuration of the end effector electrical connection assembly 210 and the tubular shaft connection assembly 230. For example, if the end effector electrical connection assembly 210 comprises first and second end effector electrical pads 214, 216 on an inwardly-facing surface of the end effector electrical connection assembly 210, the corresponding the first and second distal electrical pads 224, 226 can be disposed on the outwardly-facing surface of the body 222, and vice-versa.

FIG. 6B is a first side view while FIG. 6C is a second side view of the electrical connector 200, in accordance with the disclosed technology. To help ensure the electrical connector 200 is properly aligned with the tubular shaft 112 and the end effector 28, the body 222 can include an alignment member 223 that can be configured to align with, and be compliant with, a corresponding alignment member disposed on the end effector 28 and/or the tubular shaft 112 (not shown). The alignment member 223 can be a notch, as shown, or the alignment member 223 can be a protrusion or other similar member that can cause the end effector 28 and/or the tubular shaft 112 to be aligned with the electrical connector 200 in only one orientation. In this way, the electrical connector 200 can be configured to ensure the end effector 28 and tubular shaft 112 are aligned (or "clocked") correctly with the electrical connector 200 and that the electrical pads are all properly aligned. The electrical connector 200 can further include colors, markings, or other features that ensure the electrical connector 200 is properly aligned with the end effector 28 and tubular shaft 112 when assembled.

FIG. 6C further shows a gap 225 that can be formed in the electrical connector 200. The gap 225, for example, can be a result of the electrical connector 200 initially being formed as planar sheet and then formed into the cylindrical form shown. Furthermore, the gap 225 can permit the body 222 to flex and enable easier assembly with the end effector 28 and tubular shaft 112.

As will be appreciated by one of skill in the art, the particular arrangement of proximal electrical pads 228, first distal electrical pads 224, and the second distal electrical pads 226 of the electrical connector 200 shown in FIGs. 6A-6C are offered for illustrative purposes and various other arrangements are contemplated and are included within the scope of this disclosure. Therefore, the scope of this disclosure should not be limited to the particular arrangement of components shown and described herein.

In some examples, the electrical connector 200 can be configured such, when selected distal electrical pads 224, 226 are in electrical communication with corresponding first and second end effector electrical pads 214, 216, the corresponding proximal electrical pads 228 can similarly be in electrical communication with tubular shaft electrical pads 236 that correspond to the first and second end effector electrical pads 214, 216. That is, if an end effector 28 does not have as many electrical pads as there are electrical pads on the electrical connector, the electrical can be configured such that the electrical pads that are actually used align with, and/or electrically connected to, the corresponding tubular shaft electrical pads. That is in one operative condition, when the electrical connector 200 is connected to a tubular shaft 112 at the proximal portion and an end effector 28 and the distal portion, respective tubular shaft electrical pads 236 are in electrical communication with corresponding end effector electrical pads (214, 216) via the electrical connector 200, and corresponding proximal electrical pads 228 that are not in electrical communication with tubular shaft electrical pads 236 are aligned with distal electrical pads (224, 226) that are not in electrical communication with end effector electrical pads (214, 216).

FIG. 7A is a perspective view while FIG. 7B is a top view of the distal end of the tubular shaft 112, in accordance with the disclosed technology. As shown, the tubular shaft 112 can comprise a shaft body 232 comprising a recess 234 formed into the distal end of the shaft body 232 that can be configured to receive the electrical connector 200 and at least part of the end effector 28. The recess can be sized such that the tubular shaft 112 forms a friction fit with the electrical connector 200 and/or the end effector 28. The tubular shaft 112 can further include a lumen 238 extending therethrough. In some examples, the lumen 238 can be sized to receive a push rod, an irrigation tube, or various other components of the end effector 28 extending through the lumen 238.

As best seen in FIG. 7B, the distal end of the tubular shaft 112 can further include a plurality of tubular shaft electrical pads 236 that can be disposed radially around the tubular shaft 112. The tubular shaft electrical pads 236 can be arranged such that the tubular shaft electrical pads 236 are configured to contact and be in electrical communication with the proximal electrical pads 228.

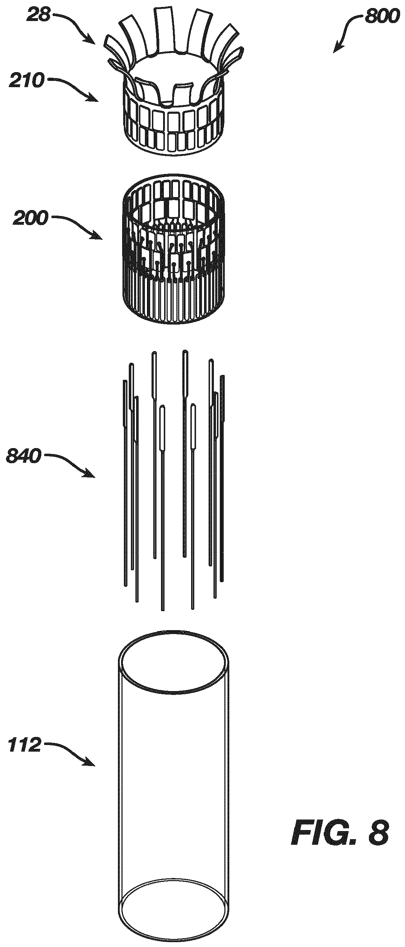

FIG. 8 is a perspective exploded view of another example medical probe 800 including a tubular shaft 112, electrical connector 200, and the end effector 28, in accordance with the disclosed technology. The medical probe 800 can comprise all of the same features as the catheter 14 previously described except that the medical probe 800 can include a plurality of wires connectors 840 instead of the recess 234 and tubular shaft electrical pads 236 previously described.

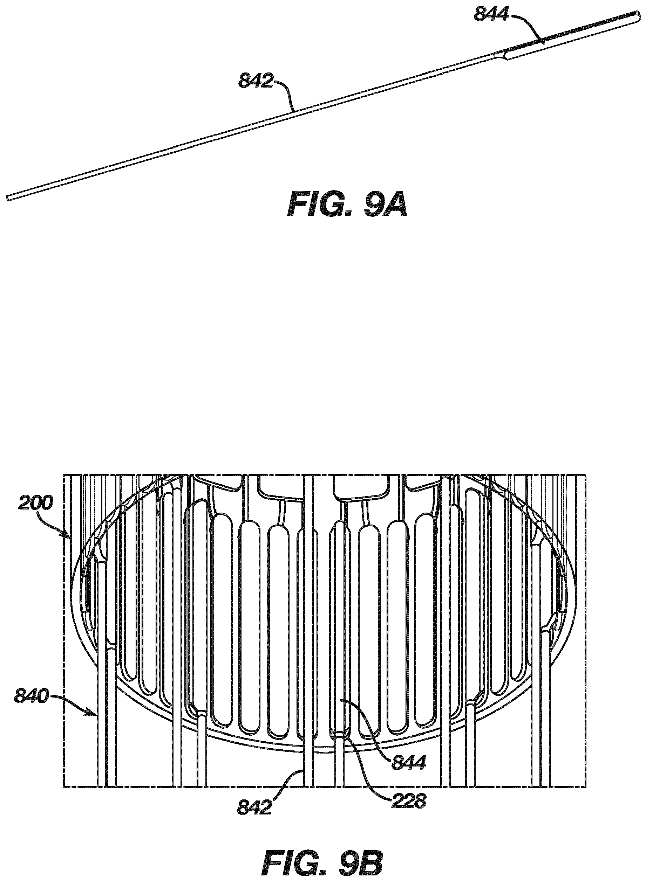

The wire connectors 840, as further shown in FIGs. 9A and 9B, can include a wire 842 and a wire electrical pad 844 disposed at a distal end of the wire 842. The proximal end of the wire 842 can be connected to the electrical connection point 130 on the handle 110. The wire electrical pads 844, as further shown in FIGs. 9A and 9B, can be configured to be connected to the proximal electrical pads 228 of the electrical connector 200. The wire electrical pads 844 can be secured within the tubular shaft 112 by a harness or other apparatus configured to keep the wire electrical pads 844 in place such that the proximal electrical pads 228 can be aligned and in contact with the wire electrical pads 844 when the electrical connector 200 is inserted into (or around) the tubular shaft 112.

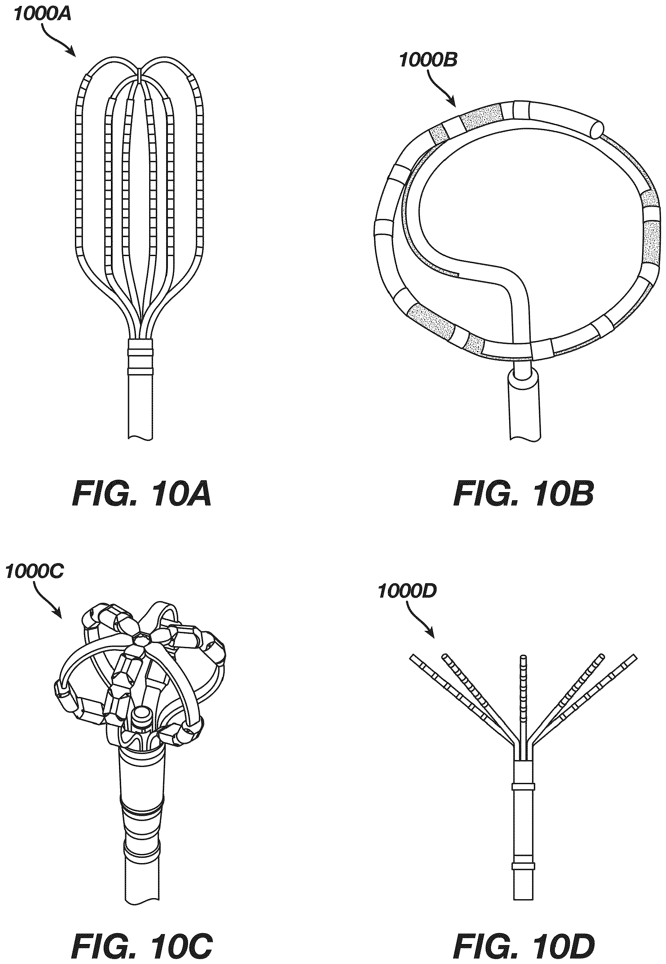

As described previously, the electrical connector 200 can be configured to be attached to a plurality of different types of end effectors. FIGs. 10A-10D illustrate various different types of end effectors that can be attached to the electrical connector 200. For example, the electrical connector can be attached to a planar array catheter 1000A (FIG. 10A), a spiral or circular catheter 1000B (FIG. 10B), a basket catheter 1000C (FIG. 10C), or catheters having spines extending outwardly such as the spine catheter 1000D shown in FIG. 10D. As will be appreciated by one of skill in the art, the various end effectors shown and described in FIGs. 10A-10D can include varying numbers, types, and arrangements of electrodes attached thereto. Furthermore, the various end effectors shown and described in FIGs. 10A-10D are not intended to be limiting and are offered solely for illustrative purposes as the electrical connector 200 can be configured to electrically connect various other types of end effectors to the tubular shaft 112. The electrical connector 200 can make it possible for each of these catheters, as well as various other catheters not shown, to be attached to the tubular shaft 112 in an easy and efficient manner.

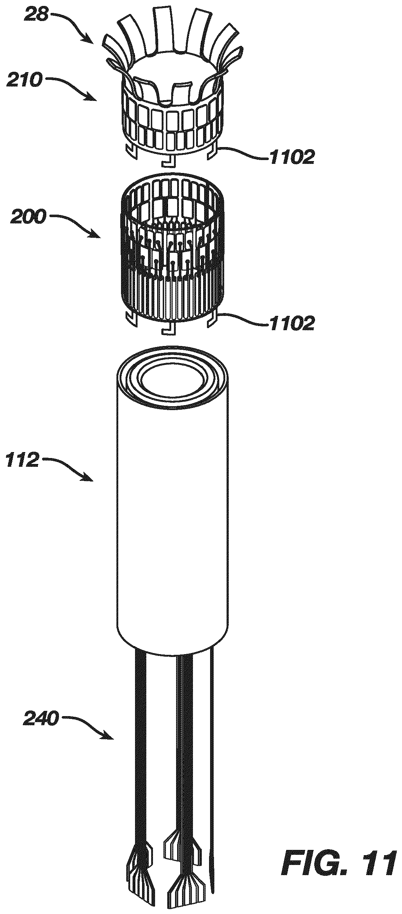

FIG. 11 is an exploded view of another example of the tubular shaft 112, the electrical connector 200, and the end effector 28, in accordance with the disclosed technology. The example shown in FIG. 11 illustrates the end effector 28 and the electrical connector 200 comprising a mechanical connector 1102 that can be configured to attach the end effector 28 to the electrical connector 200 and/or the tubular shaft 112 and to attach the electrical connector 200 to the tubular shaft 112. The mechanical connectors 1102 are shown as being on a proximal side of the electrical connector 200 and the end effector 28, however, it should be appreciated by one of skill in the art that the mechanical connectors 1102 can be disposed on the distal side of the tubular shaft 112 and/or the electrical connector 200 instead of, or in addition to, being on the proximal side of the electrical connector 200 and the end effector 28.

The mechanical connectors 1102 can be configured to interlock with corresponding mechanical connectors (not shown) on the tubular shaft 112, the electrical connector 200, and/or the end effector 28. The mechanical connectors 1102 can be or include a bayonet mount, a snap fitting, a threaded connector, or other similar mechanical connectors that can attach the components together. The mechanical connectors 1102 can be in place of, or in addition to, the alignment members previously described. Furthermore, the number of mechanical connectors 1102 can be varied depending on the particular application. For example, a single mechanical connector 1102 can be included or multiple mechanical connectors 1102 can be included. In other examples, an adhesive can be used to connect the tubular shaft 112, electrical connector 200, and end effector 28 to each other.

The disclosed technology described herein can be further understood according to the following clauses:
Clause 1: an electrical connector for a catheter device comprising: a body extending along a longitudinal axis from a proximal portion to a distal portion, the proximal portion configured to be attached to a tubular shaft and the distal portion configured to be attached to an end effector; a plurality of proximal electrical pads disposed on the body at the proximal portion, the proximal electrical pads configured to be in electrical communication with a plurality of tubular shaft electrical pads; a plurality of distal electrical pads disposed on the body at the distal portion, the distal electrical pads configured to be in electrical communication with a plurality of end effector electrical pads; and a plurality of electrical traces, each electrical trace connecting a respective proximal electrical pad to a respective distal electrical pad.
Clause 2: The electrical connector according to clause 1, the body comprising a generally cylindrical shape.
Clause 3: The electrical connector according to clauses 1 or 2, the plurality of proximal electrical pads being disposed on an outer surface of the body.
Clause 4: The electrical connector according to clause 3, the plurality of proximal electrical pads being spaced along the outer surface of the body in a longitudinal and radial direction.
Clause 5: The electrical connector according to clause 1 or clause 2, the body defining a lumen extending therethrough.
Clause 6: The electrical connector according to clause 5, the plurality of proximal electrical pads being disposed on an inner surface of the body in the lumen.
Clause 7: The electrical connector according to clause 6, the plurality of proximal electrical pads being spaced along the inner surface of the body in a longitudinal and radial direction.
Clause 8: The electrical connector according to any of the preceding clauses, the plurality of distal electrical pads being disposed on an outer surface of the body.
Clause 9: The electrical connector according to clause 8, the plurality of distal electrical pads being spaced along the outer surface of the body in a longitudinal and radial direction.
Clause 10: The electrical connector according to any of clauses 5-7, the plurality of proximal electrical pads being disposed on the inner surface of the body in the lumen.
Clause 11: The electrical connector according to clause 10, the plurality of distal electrical pads being spaced along the inner surface of the body in a longitudinal and radial direction.
Clause 12: The electrical connector according to any of the preceding clauses, the body being generally tapered toward the proximal portion along the longitudinal axis.
Clause 13: The electrical connector according to any of clauses 1-11, the body being generally tapered toward the distal portion along the longitudinal axis.
Clause 14: The electrical connector according to any of the preceding clauses, the body further comprising a proximal connector, the proximal connector configured for mechanically attaching the body to the tubular shaft.
Clause 15: The electrical connector according to clause 14, the proximal connector comprising a bayonet mount.
Clause 16: The electrical connector according to clause 14, the proximal connector comprising a threaded connector.
Clause 17: The electrical connector according to any of the preceding clauses, the body further comprising a proximal alignment member configured to align with a corresponding alignment member on the tubular shaft.
Clause 18: The electrical connector according to any of the preceding clauses, the body further comprising a distal connector, the distal connector configured for mechanically attaching the body to the end effector.
Clause 19: The electrical connector according to clause 18, the distal connector comprising a bayonet mount.
Clause 20: The electrical connector according to clause 18, the distal connector comprising a threaded connector.
Clause 21: The electrical connector according to any of the preceding clauses, the body further comprising a distal alignment member configured to align with a corresponding alignment member on the end effector.
Clause 22: The electrical connector according to any of clauses 17-21, wherein when the electrical connector is connected to a tubular shaft at the proximal portion and an end effector and the distal portion, respective tubular shaft electrical pads are in electrical communication with corresponding end effector electrical pads via the electrical connector, and corresponding proximal electrical pads that are not in electrical communication with tubular shaft electrical pads are aligned with distal electrical pads that are not in electrical communication with end effector electrical pads.
Clause 23: A medical probe, comprising: a tubular shaft extending along a longitudinal axis of the medical probe; an end effector comprising a plurality of electrodes; and an electrical connector disposed between the tubular shaft and the end effector, the electrical connector comprising: a body extending along the longitudinal axis from a proximal portion to a distal portion; a plurality of proximal electrical pads disposed on the body at the proximal portion, the proximal electrical pads configured to align with, and electrically connect to, a plurality of tubular shaft electrical pads disposed on the tubular shaft; a plurality of distal electrical pads disposed on the body at the distal portion, the distal electrical pads configured to align with, and electrically connect to, a plurality of end effector electrical pads disposed on the end effector; and a plurality of electrical traces, each electrical trace connecting a respective proximal electrical pad to a respective distal electrical pad.
Clause 24: The medical probe according to clause 23, the electrical connector further comprising a proximal connector, the proximal connector configured for mechanically attaching the body to the tubular shaft.
Clause 25: The medical probe according to clause 23, the electrical connector further comprising a distal connector, the distal connector configured for mechanically attaching the body to the end effector.
Clause 26: The medical probe according to any of clauses 23-25, the tubular shaft further comprising a recess configured to at least partially receive the electrical connector.
Clause 27: The medical probe according to clause 26, wherein the tubular shaft electrical pads are disposed in the recess.
Clause 28: The medical probe according to any of clauses 23-27, the electrical connector further comprising a proximal alignment member configured to align with a corresponding alignment member on the tubular shaft.
Clause 29: The medical probe according to any of clauses 23-28, the electrical connector further comprising a distal alignment member configured to align with a corresponding alignment member on the end effector.
Clause 30: The medical probe according to any of clauses 28 and 29, wherein in one operative condition, the electrical connector is connected to the tubular shaft at the proximal portion and an end effector and the distal portion, respective tubular shaft electrical pads are in electrical communication with corresponding end effector electrical pads via the electrical connector, and corresponding proximal electrical pads that are not in electrical communication with tubular shaft electrical pads are aligned with distal electrical pads that are not in electrical communication with end effector electrical pads.
Clause 31: The medical probe according to any of clauses 23-30, further comprising an adhesive configured bond the tubular shaft to the electrical connector.
Clause 32: The medical probe according to any of clauses 23-31, further comprising an adhesive configured bond the end effector to the electrical connector.
Clause 33: The medical probe according to any of clauses 23-32, at least one of the plurality of electrodes being configured for detecting one or more electrophysiological signals.
Clause 34: The medical probe according to any of clauses 23-32, at least one of the plurality of electrodes being configured to emit energy for ablation of tissue.
Clause 35: The medical probe according to any of clauses 23-34, the end effector comprising a basket catheter.
Clause 36: The medical probe according to any of clauses 23-34, the end effector comprising a planar catheter.
Clause 37: The medical probe according to any of clauses 23-34, the end effector comprising a plurality of arms extending radially outward from the longitudinal axis.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An electrical connector for a catheter device comprising:
a body extending along a longitudinal axis from a proximal portion to a distal portion, the proximal portion configured to be attached to a tubular shaft and the distal portion configured to be attached to an end effector;
a plurality of proximal electrical pads disposed on the body at the proximal portion, the proximal electrical pads configured to be in electrical communication with a plurality of tubular shaft electrical pads;
a plurality of distal electrical pads disposed on the body at the distal portion, the distal electrical pads configured to be in electrical communication with a plurality of end effector electrical pads; and
a plurality of electrical traces, each electrical trace connecting a respective proximal electrical pad to a respective distal electrical pad.

2. The electrical connector according to claim 1, the plurality of proximal electrical pads being spaced along an outer surface of the body in a longitudinal and radial direction, optionally the plurality of distal electrical pads being spaced along the outer surface of the body in a longitudinal and radial direction.

3. The electrical connector according to claim 1, the body defining a lumen extending therethrough.

4. The electrical connector according to claim 3, the plurality of proximal electrical pads being spaced along an inner surface of the body in a longitudinal and radial direction.

5. The electrical connector according to claim 4, the plurality of distal electrical pads being spaced along the inner surface of the body in a longitudinal and radial direction.

6. The electrical connector according to claim 1, the body being generally tapered toward the proximal portion along the longitudinal axis, or generally tapered toward the distal portion along the longitudinal axis.

7. The electrical connector according to claim 1, the body further comprising a proximal connector, the proximal connector configured for mechanically attaching the body to the tubular shaft, optionally the proximal connector comprising a bayonet mount.

8. The electrical connector according to claim 1, the body further comprising a proximal alignment member configured to align with a corresponding alignment member on the tubular shaft.

9. The electrical connector according to claim 1, the body further comprising a distal connector, the distal connector configured for mechanically attaching the body to the end effector, optionally the distal connector comprising a bayonet mount.

10. The electrical connector according to claim 1, the body further comprising a distal alignment member configured to align with a corresponding alignment member on the end effector.

11. The electrical connector according to claim 1, wherein when the electrical connector is connected to a tubular shaft at the proximal portion and an end effector at the distal portion, respective tubular shaft electrical pads are in electrical communication with corresponding end effector electrical pads via the electrical connector, and corresponding proximal electrical pads that are not in electrical communication with tubular shaft electrical pads are aligned with distal electrical pads that are not in electrical communication with end effector electrical pads.

12. A medical probe, comprising:
a tubular shaft extending along a longitudinal axis of the medical probe;
an end effector comprising a plurality of electrodes; and
an electrical connector disposed between the tubular shaft and the end effector, the electrical connector comprising:
a body extending along the longitudinal axis from a proximal portion to a distal portion;
a plurality of proximal electrical pads disposed on the body at the proximal portion, the proximal electrical pads configured to align with, and electrically connect to, a plurality of tubular shaft electrical pads disposed on the tubular shaft;
a plurality of distal electrical pads disposed on the body at the distal portion, the distal electrical pads configured to align with, and electrically connect to, a plurality of end effector electrical pads disposed on the end effector; and
a plurality of electrical traces, each electrical trace connecting a respective proximal electrical pad to a respective distal electrical pad.

13. The medical probe according to claim 12, the electrical connector further comprising:
a proximal connector, the proximal connector configured for mechanically attaching the body to the tubular shaft; and
a distal connector, the distal connector configured for mechanically attaching the body to the end effector.

14. The medical probe according to claim 12 or claim 13, the tubular shaft further comprising a recess configured to at least partially receive the electrical connector, optionally wherein the tubular shaft electrical pads are disposed in the recess.

15. The medical probe according to claim 14, wherein in one operative condition, the electrical connector is connected to the tubular shaft at the proximal portion and an end effector at the distal portion, respective tubular shaft electrical pads are in electrical communication with corresponding end effector electrical pads via the electrical connector, and corresponding proximal electrical pads that are not in electrical communication with tubular shaft electrical pads are aligned with distal electrical pads that are not in electrical communication with end effector electrical pads.
